# EUROPEAN PATENT SPECIFICATION

(11) **EP 4 729 118 B1**
(45) Date of publication and mention of the grant of the patent: **14.10.2026**
(21) Application number: 24207257.7
(22) Date of filing: 17.10.2024
(51) Int. Cl.: A61N 5/06

(54) **MEDICAL HEAD COVERING**
MEDIZINISCHE KOPFBEDECKUNG
COUVRE-CHEF MÉDICAL

(43) Date of publication of application: 22.04.2026
(73) Proprietor: Weber Medical Systems GmbH, 37697 Lauenförde (DE)
(72) Inventor: DJIE, Eric, 1016RP Amsterdam (NL)
(74) Representative: Lüdtke, Thomas

(56) References cited:
- US-A1- 2020 360 715
- US-A1- 2023 185 096
- KHERADMAND ALI ET AL: "Effect of low-level laser therapy (LLLT) on cognitive impairment among patients with chronic schizophrenia: a double-blind randomized placebo-controlled clinical trial", LASERS IN MEDICAL SCIENCE, SPRINGER LONDON, LONDON, vol. 37, no. 6, 22 March 2022 (2022-03-22), pages 2717 - 2725, XP037920393, DOI: 10.1007/S10103-022-03545-9

## Description

The present invention relates to a medical head covering, according to claim 1.

Transcranial laser therapy (TLT), also known as photobiomodulation (PBM) when referring to its biological effects, is a non-invasive therapeutic technique that uses low-level laser light or near-infrared light to stimulate the brain. Its roots lie in the broader field of light therapy, which dates back to the early 20th century when researchers discovered that certain wavelengths of light could have beneficial effects on biological tissues. The specific use of light therapy to stimulate brain tissue began to develop more fully in the late 20th century, following advances in laser technology and the growing understanding of how light can interact with living cells. Early studies showed that light could penetrate biological tissues and have therapeutic effects, such as reducing inflammation, stimulating cellular repair processes, and enhancing blood flow.

The concept of applying light to the brain to treat neurological conditions emerged as research on low-level laser therapy (LLLT) expanded. In the 1960s and 1970s, lasers were first used in medical treatments, particularly in dermatology and wound healing. It was during this time that researchers began exploring whether light could penetrate the skull to reach the brain. Although the skull does block much light, particularly visible light, researchers found that near-infrared (NIR) light, with wavelengths between 800 and 1100 nanometers, could penetrate the skull and potentially reach the brain. By the 1990s and 2000s, transcranial laser therapy began to be explored more seriously as a treatment for various neurological conditions. Pioneering work by researchers such as Dr. Michael Hamblin at Harvard University and others helped define the mechanisms by which light affects cellular function, particularly by acting on the mitochondria (the energy-producing components of cells) to enhance cellular respiration and energy production. These discoveries fueled interest in using TLT to treat neurological injuries and diseases.

Transcranial laser therapy works primarily through a process known as photobiomodulation. When near-infrared light penetrates the scalp and skull, it reaches brain tissue, where it is absorbed by chromophores, specifically cytochrome c oxidase in the mitochondria. This absorption enhances mitochondrial function, leading to increased adenosine triphosphate (ATP) production, which is essential for cellular energy. This improved cellular energy status can have several beneficial effects, including:
Increased neurogenesis: The creation of new neurons and support cells. Enhanced synaptic plasticity: Improving the connections between neurons, which is crucial for learning and memory.

Reduction of inflammation: Light therapy can decrease inflammatory markers, reducing damage to neurons in certain conditions.

Improved cerebral blood flow: Better blood flow can help deliver more oxygen and nutrients to brain tissues.

Decreased oxidative stress: Photobiomodulation reduces oxidative damage to cells by enhancing antioxidant defenses.

Transcranial laser therapy has been studied and applied to a range of neurological conditions and cognitive performance areas, with varying degrees of clinical validation. Some of the most prominent applications include:
One of the earliest and most promising applications of TLT is the treatment of traumatic brain injury. TBI can cause widespread damage to brain cells due to inflammation, reduced blood flow, and oxidative stress. Studies have shown that TLT can help reduce inflammation, promote neurogenesis, and improve recovery after TBI. Clinical trials have demonstrated improvements in cognitive function, memory, and overall neurological health in patients who received TLT following brain injuries.

TLT has been studied as a potential therapy to promote recovery in stroke patients. Stroke leads to brain damage due to the interruption of blood flow, resulting in oxygen deprivation to brain tissue. TLT has shown potential in improving cerebral blood flow and reducing tissue damage by stimulating cellular repair processes and reducing inflammation. Clinical trials, such as those by Dr. Margaret Naeser and colleagues, have shown promising results in stroke rehabilitation, especially when applied in the acute stages of stroke recovery.

Neurodegenerative diseases such as Alzheimer's disease and Parkinson's disease involve progressive damage to neurons over time. TLT has been proposed as a therapy to slow down disease progression by promoting neuronal survival, reducing oxidative stress, and enhancing synaptic plasticity. While research is still in the early stages, preliminary studies have shown improvements in cognitive function and motor skills in patients with Alzheimer's and Parkinson's who have undergone TLT treatment.

Transcranial laser therapy has also been investigated as a treatment for mood disorders, including major depressive disorder. Several studies have demonstrated that TLT applied to the prefrontal cortex (an area associated with mood regulation) can alleviate symptoms of depression and anxiety. This effect is likely due to enhanced synaptic plasticity, improved blood flow, and reduction of inflammation in brain regions that regulate emotion. Clinical trials have reported positive outcomes in patients who were resistant to conventional treatments such as medication and psychotherapy.

In addition to therapeutic applications, TLT has been explored for its potential to enhance cognitive performance in healthy individuals. Some studies have found that TLT can improve attention, memory, and executive function by stimulating neurogenesis and increasing blood flow to key brain regions. This application is of particular interest in the context of age-related cognitive decline, where TLT might help improve brain function in older adults.

TLT has also been studied as a non-invasive option for reducing the frequency and intensity of migraines and headaches. Its ability to reduce inflammation and improve blood flow may help alleviate pain and other symptoms associated with chronic migraines.

Although transcranial laser therapy is still in the early stages of widespread clinical adoption, research is continuing to support its potential across various neurological applications. Several small-scale clinical trials have demonstrated its safety and efficacy in conditions such as traumatic brain injury, stroke recovery, and mood disorders, but larger, randomized controlled trials are necessary to confirm these findings and establish standardized protocols for its use.

Several prior art systems have been developed to deliver transcranial laser therapy, with devices typically using wavelengths in the near-infrared (NIR) spectrum, such as 800-1100 nm, which has been shown to effectively penetrate biological tissues. For example, methods of treating stroke by applying laser energy to the head within 24 hours after the onset of stroke symptoms are known. The laser device emits light within the NIR range to stimulate neural tissue and improve outcomes in ischemic stroke patients. However, limitations in power delivery, treatment area coverage, and variability in patient response remain challenges in this approach.

Other systems focus on treating mood disorders such as depression through low-level laser therapy (LLLT). Exemplary devices target specific areas of the prefrontal cortex with low-intensity light, aiming to stimulate neural activity and improve mood. While this method shows promise, there are concerns about the precision of light delivery and the optimization of dosage parameters, which can vary significantly between patients.

Similarly, a helmet-based laser therapy device designed to deliver photonic energy to the brain in a diffuse manner is known. This device covers a larger surface area of the skull, allowing for a more generalized treatment approach. Despite these advances, current helmet-based systems may suffer from uneven light distribution across the brain's surface, leading to suboptimal treatment efficacy in certain regions.

While prior technologies have demonstrated the potential benefits of TLT, they are often limited by challenges such as insufficient light penetration, difficulty in targeting specific brain regions, and inconsistent results across different patients. Furthermore, safety concerns related to thermal effects and prolonged exposure to laser energy have hindered the widespread adoption of these devices.

One specific problem encountered in transcranial laser therapy (TLT) is the blockage or attenuation of light caused by the presence of hair on the patient's scalp. Hair, particularly dark or thick hair, absorbs and scatters laser light, reducing the amount of light that penetrates the scalp and reaches the underlying brain tissue. This can significantly diminish the therapeutic effectiveness of TLT, as insufficient light energy may be delivered to the target regions of the brain. Hair acts as a barrier by absorbing light, especially in the near-infrared (NIR) spectrum, which is commonly used in TLT. Darker hair, which contains higher concentrations of melanin, has a greater tendency to absorb light, further limiting the energy that can pass through to the scalp. Additionally, the density and thickness of the hair can scatter the light, causing it to diffuse in unintended directions before it reaches the skin. This issue introduces variability in treatment outcomes among patients, as the degree of light blockage varies depending on the individual's hair characteristics.

To address this problem, several approaches have been attempted in prior art, including requiring patients to shave the treatment area or use compression devices to flatten the hair against the scalp. However, these solutions are inconvenient, uncomfortable, or impractical for regular therapeutic use, especially in at-home or long-term treatments.

Another significant challenge in current transcranial light therapy (TLT) systems is the difficulty in designing devices that are easy to use and effective for a wide variety of patients with different head sizes and shapes. The anatomical diversity in head size, skull shape, and scalp contour can affect how the light therapy device fits and delivers treatment, potentially reducing its efficacy and limiting its usability. Existing transcranial laser therapy devices, such as helmets or headbands, are often designed with a fixed or semi-adjustable structure. These designs may not conform well to the unique geometry of each patient's head, leading to several issues:
A device that does not conform closely to the patient's head may result in uneven contact between the light-emitting surface and the scalp. This can lead to inconsistent light delivery across the treatment area, with some regions receiving suboptimal dosages of light, while other areas may be exposed to excessive energy. This inconsistency diminishes the overall therapeutic effectiveness of the treatment. When a device does not properly fit the contour of the head, gaps can form between the device and the scalp, allowing light to leak out. This not only reduces the amount of light penetrating the skin but also poses a potential safety concern due to unintended light exposure to the patient's eyes or the surrounding environment.

Patients with larger or smaller heads may experience discomfort with ill-fitting devices, causing pressure points or difficulty maintaining a secure fit during treatment sessions. This discomfort can lead to poor compliance with prescribed therapy, especially for treatments requiring regular, long-term use, wherein patient's treatment compliance is one of the biggest challenges to a positive outcome of any therapy. Variability in head shapes and sizes also impacts the precision with which certain brain regions can be targeted. Current devices may struggle to align correctly with specific areas of the brain in patients with particularly large, small, or irregularly shaped heads, leading to reduced efficacy in targeting critical regions such as the prefrontal cortex or motor regions.

Further, a significant problem with current transcranial light therapy (TLT) systems is the buildup of heat during treatment. Transcranial laser devices typically use light in the near-infrared (NIR) spectrum, which, while effective at penetrating the scalp and skull to reach brain tissues, can also generate heat as the light interacts with biological tissues. This heat buildup presents several challenges that impact both the safety and efficacy of the treatment:
Excessive heat generated during transcranial light therapy can pose a risk of thermal damage to the scalp and underlying tissues. Although TLT devices are designed to operate within safe parameters, prolonged exposure or highintensity treatments may cause localized heating, which can lead to discomfort, skin irritation, or even burns in extreme cases. This concern is particularly relevant in treatments involving prolonged sessions or higher power densities to achieve therapeutic effects.

The buildup of heat during treatment may prompt the body's natural thermoregulatory response, including increased blood flow to the scalp to dissipate heat. This increased perfusion can result in unwanted changes in the distribution of light energy, potentially reducing the precision and effectiveness of targeting specific brain regions. Furthermore, excessive heat may cause patient discomfort, leading to shortened treatment sessions or the need to reduce laser intensity, compromising the overall therapeutic outcome.

Even at moderate power levels, patients may experience discomfort due to localized heating during TLT. This discomfort can manifest as a feeling of warmth, burning, or general irritation on the scalp, making it difficult for patients to tolerate the full duration of the treatment. For therapies requiring regular sessions, this discomfort may reduce patient compliance, leading to inconsistent treatment and suboptimal results.

To mitigate heat buildup, some systems incorporate active cooling mechanisms, such as fans, heat sinks, or liquid cooling systems, to dissipate excess heat during treatment. However, these solutions add complexity and cost to the devices, increase their size and weight, and may introduce noise or other operational issues. Additionally, these cooling systems may not always be effective in uniformly preventing heat buildup across the entire treatment area, especially in devices that cover large regions of the scalp.

The buildup of heat can also alter the optical properties of the skin and scalp, affecting how light is absorbed or scattered during treatment. For example, heating may lead to changes in tissue hydration or blood flow, which could modify the light penetration characteristics and reduce the consistency of the therapeutic dose delivered to deeper brain regions.

Further, it is known that treatment of androgenetic alopecia with red light therapy with Minoxidil or Finasteride yields better results in increasing hair count than either of the two treatments on its own. The best and most effective approach is to start with the treatment as early as possible when there is still plenty of hair. The main problem with such a preventive treatment with red light therapy is that the still present hair blocks the light to reach the hair follicles especially for those with darker hair. Red light, wavelengths around 650nm, are well described for treatment of early hair loss androgenetic alopecia. But in the early stages of hair loss, the still present hair blocks the light from reaching the scalp. Hence currently existing red light therapy devices have limited efficacy.

US 2023/185096 A1 discloses a head covering configured for placement and size adjustment via circumferential and sagittal bands or straps that include length- and tension-adjusters to tighten or loosen the frame to a user's head. Further, functional modules are mounted on a tessellated array of polygonal tiles or attachment points that allow functional modules to be repositioned (e.g., moved between tiles or along guided features) for subject-specific targeting.

US 2020/360715 A1 describes a head covering formed by intersecting straps joined at nodes to create a cap-like scaffold. Length-adjustable strap segments provide global fit. Independent optoelectronic modules (emitters/detectors) are mounted on sliding, lockable carriers along the straps and include local tilt/rotation and standoff/compliant interfaces to set incidence angle and source-detector spacing for different scalp regions.

A further medical head covering is described in "Effect of low-level laser therapy (LLLT) on cognitive impairment among patients with chronic schizophrenia: a double-blind randomized placebo-controlled clinical trial" LASERS IN MEDICAL SCIENCE, SPRINGER LONDON, LONDON, vol. 37, no. 6, 22 March 2022 (2022-03-22), pages 2717-2725, XP037920393, DOI: 10.1007/S10103-022-03545-9.

To address all these problems, improvements in such light therapy systems are needed. It is therefore object of the present invention to provide a medical head covering that optimizes therapeutical and/or diagnostic treatment, in particular, heat generation ensuring patient comfort without adding complexity and, simultaneously, allows a uniform distribution of light energy across the treatment area for delivering targeted, effective and safe treatments across a broad range of neurological conditions, as well as enabling to accommodate a wide range of head sizes and shapes without sacrificing comfort or efficacy in regular therapeutic and/or diagnostic use.

This objective is solved by a medical head covering of the invention as described in independent claim 1. Advantageous embodiments of the invention are described in the subclaims.

Thus, according to the invention, a medical head covering for the treatment and/or diagnosis of a user's head is provided, comprising:
- functional modules, e.g., containing diodes or electrodes or the like, for the treatment and/or diagnosis of the user's head, once the medical head covering is put onto the user's head; and
- a super structure comprising at least three essentially semi-circular rigid elastic elements joined together in a joining area so as to partially enclose an interior space having a curved surface for accommodating the head of the user, wherein
   --- at least a front rigid elastic element that is extending in a front part of the medical head covering and at least one rear rigid elastic element that is extending in a rear part of the medical head covering are joined together in the joining area so as to form a rigid and elastic circle or ellipse around the interior space, and
   --- at least one upper rigid elastic element is extending in an upper part of the medical head covering and partly enclosing the interior space from above, e.g., joined to the other rigid elastic elements (front, rear) in the joining area,
whereby the functional modules are connected to the super structure individually or in groups via mounting elements, either rigidly or movably, in such a way that they are oriented into the interior space for approaching or touching the user's head, in particular, during therapeutical and/or diagnostic use.

Advantageously, with such a medical head covering a convenient, comfortable, and practical medical device for a regular therapeutic and/or diagnostical use, especially in at-home or long-term treatments, is provided, that overcomes the disadvantages of the prior art systems described above. In particular, the inventive solution allows for a customized fit for each patient regardless of anatomical differences, ensuring consistent (over the whole device) approaching or touching of the functional elements to the scalp of the user, as the individual rigid elastic elements of the super-structure that are distributed over the front, rear and upper part of the head covering are flexible and can adjust to the shape of the user's scalp very easily and comfortably.

Thereby, rigid elastic element is to be understood as an element that in its essentially semi-circular form is stiff and has the flexibility and elasticity constrained to a single plane, wherein it can bend under external forces, store potential energy, and return to their original shape (neutral position), preferably to a semi-circular shape, upon the removal of those forces. In a preferred embodiment, the rigid elastic elements each comprise or are made of at least one blade spring with a neutral position or free state in a semi-circular shape, wherein the at least one blade spring is preferably made of metal. As the super structure comprises three of these semicircular rigid elastic elements joined in the joining area and assembled in a three-dimensional or spherical configuration it is ensured that the medical therapy head covering has a structural robustness that allows for ease of handling simultaneously allowing a comfortable seat on the user's head due to its remaining flexibility. At the same time, the robust structure allows the functional elements mounted to it to make sure that the functional elements can be well positioned on the head and relative to the surface of the scalp, providing an increased therapeutical and/or diagnostical benefit.

According to the invention, the rigid elastic elements are each joined together in a left joining area in a left part of the medial head covering and in a right joining area in a right part of the medical head covering, respectively, wherein the left part and the right part are located opposite to each other within the medical head covering, e.g., above the ears of the user once put on the head, allowing an easy and robust structure that is good to handle.

According to the invention, the rigid elastic elements each comprise a sliding mechanism for individually adjusting the length of the respective rigid elastic element for flexible adaptation of the interior space to the shape and size of the head of the user, wherein the respective sliding mechanism preferably comprises a guiding part, wherein the at least one blade spring is at least partially, e.g., with its end section, movably accommodated and guided by said guiding part such that the at least one blade spring can slidingly move against the respective guiding part for allowing to slidingly extend and retract the respective rigid elastic element.

This allows for a further adjustment of the interior space to the different shapes of a user's head providing more flexibility in use. This also allows for more comfort when putting on the head covering, as the volume of the interior space can be adjusted for a tight seat once the head covering is put on the head. Preferably, the respective sliding mechanism might comprise a pulling spring connecting the at least one blade spring and the respective guiding part in a springy manner preloading the respective rigid elastic element into a retracted position, ensuring more user comfort.

According to the invention, the rigid elastic elements are joined together in the respective joining area via hinge connections, wherein each hinge connection receives one end of the at least three rigid elastic elements such that each of the at least three rigid elastic elements can pivot against each other in the respective joining area, in particular, each of the at least three rigid elastic elements can pivot around a pivot axis extending perpendicular to the semi-circular propagation direction of the respective rigid elastic element to allow for adaptation to different shapes of the users head. These hinge connections on the sides of the super structure enable the three-dimensional spherical structure of the light therapy head covering to additionally bend in lateral direction to accommodate for different shapes of the heads of users in the interior space providing an additional level of flexibility, modularity, and comfort.

According to a preferred embodiment of the invention, the functional modules are diode patches, e.g., having an essential hexagonal shape, each having at least three, preferably six, diode systems, e.g., being located on the corners of a hexagon, with diodes emitting light along an optical axis that is defined by a lens system of the respective diode system, wherein the diodes of the diode systems of the respective diode patch are preferably configured to emit laser light or LED-light having a wavelength of approx.. 650nm for therapeutical treatment of androgenetic alopecia or between 800nm and 1100nm for the therapeutic stimulation of the brain via transcranial laser therapy, for providing a light therapy head covering as medical head covering. Thus, using the super structure for approaching or bringing the diode patches into touch with the scalp of the user provides a favorable solution for light treatment of the user's head, ensuring a customized fit for each patient and a consistent light delivery to the intended brain regions regardless of anatomical differences. However, also other functional modules, such as electrodes, can be used, providing the same advantages concerning a customized fit for each patient and a consistent contact or touch regardless of anatomical differences.

According to preferred embodiment of the invention, the diode patches comprise a back housing part forming the backside of the respective diode patch, wherein the back housing part contains or is made of a heat conductive material, e.g., aluminum alloy 1050A, and wherein the diode patches comprise a patch circuit board, wherein the diodes of the respective diode systems are electronically connected to the patch circuit board, wherein a back side of the patch circuit board is preferably made of a heat conductive material, e.g., aluminum alloy 1050A, or contains such a heat conductive material. This allows for an effective dissipation of heat, away from the head of the user, providing more comfort and safety.

According to a preferred embodiment of the invention, the respective functional modules are springy, i.e., flexible or movable against each other, combined within group elements, wherein each group element comprises at least three of said functional modules, e.g. diode patches or electrodes, that are spaced apart from each other and that are springy connected to each other via a supporting element, wherein the supporting element has at least three springy (or flexible) arms protruding in different directions, wherein each springy arm is connected to one of the at least three functional modules for springy carrying the respective functional module. This springy or flexible connection allows a good fit of the respective functional elements to the scalp of the user as the springy arms can adapt to the form of the user's head improving the diagnostic and/or therapeutical use, but also ensuring a fit that is not too tight, improving the comfort.

According to another embodiment of the invention, at least one of the diode patches comprises a proximity sensor system directed to the interior space, wherein an electronic control unit of the medical head covering, i.e., the light head covering, is electronically connected to the proximity sensor system and is adapted to stop the diodes that are assigned to this proximity sensor system from emitting light once it is determined that a distance between the nearest object in a detection area of the respective proximity sensor system is above a predetermined distance-threshold. This improves the safety, as the diodes can be stopped from emitting light, if the diode patches are located in front of the eyes of the user, for example.

### Brief description of the drawings

- Fig. 1: shows a perspective view of a light therapy head covering according to an embodiment of the invention;
- Fig. 2: shows a detailed view of the light therapy head covering without a super-structure;
- Fig. 3: shows an exploded view of a diode patch of the light therapy head covering of Fig. 1;
- Fig. 4, 5, 6: show different perspective views of a left or right patch arrangement containing several diode patches of Fig. 3;
- Fig. 7, 8: show different perspective views of a front patch arrangement containing several diode patches of Fig. 3; and
- Fig. 9, 10, 11: detailed views of a super structure of the light therapy head covering of Fig. 1.

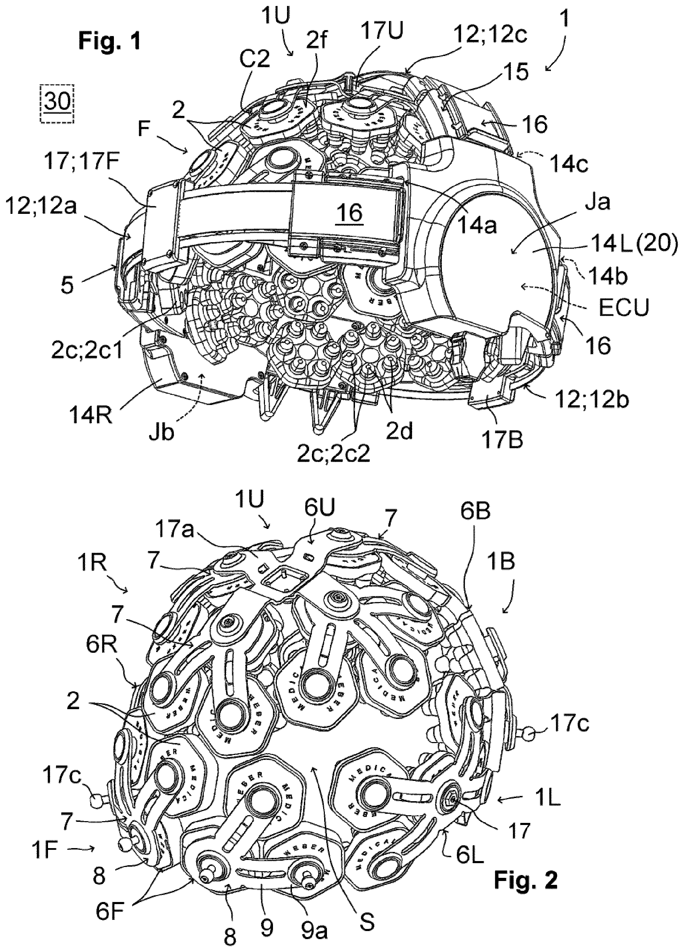

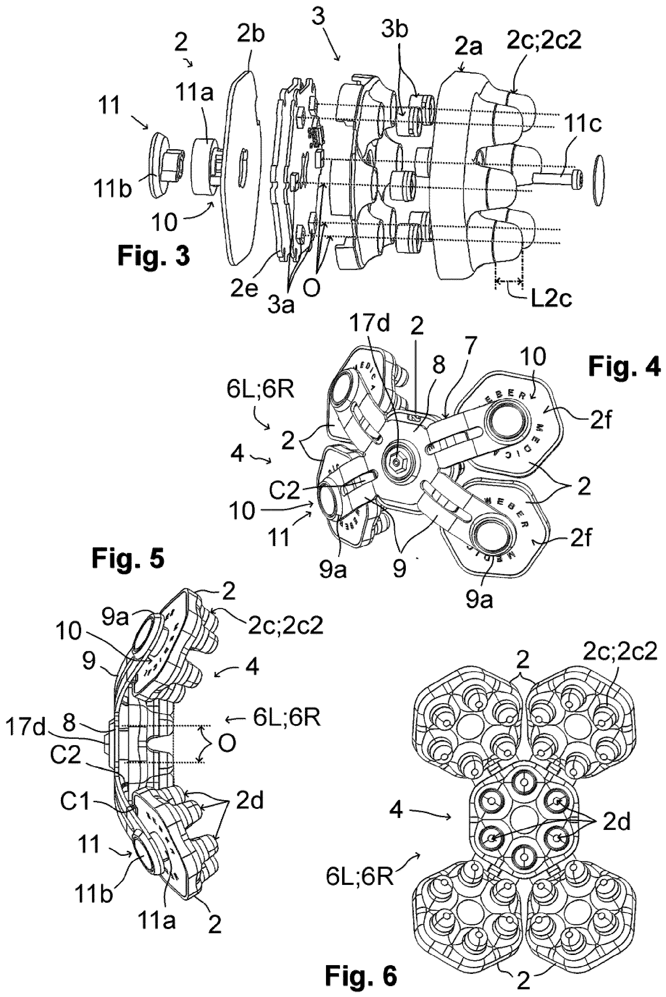

Figure 1 shows a head worn medical head covering 1 that in the shown embodiment is formed as a light therapy head covering 1a generally comprising three structural levels: The innermost level is represented by diode patches 2 as functional modules F, shown in more detail in Figs. 2 to 8 in different perspective views. The diode patches 2 each comprise several diode systems 3 each consisting of a diode 3a and a lens system 3b defining an optical axis O, as shown in Fig. 3, wherein the diode systems 3 are firmly arranged to each other in a small area, e.g. around 1100 mm², within the respective diode patch 2. In other embodiments, the functional modules F may be formed as or may comprise electrodes for diagnostic use or other elements for the treatment and/or the diagnosis of the user's head.

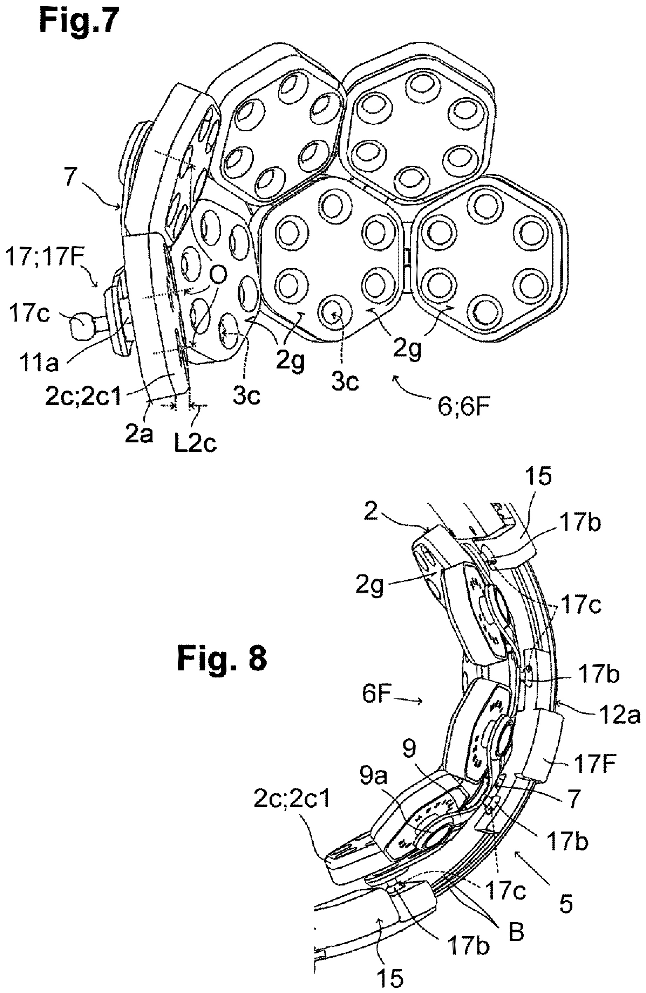

The second structural level is represented by group elements 4, wherein each group element 4 comprises several functional modules F, i.e., diode patches 2 in this embodiment, arranged and grouped together in such a way that the diode patches 2 that are combined into a certain group element 4 can move against each other offering flexibility to accommodate different shapes of scalps of users. The third and outermost structural level is represented by a super structure 5, shown in more detail in Figs. 8 to 11, wherein the super structure 5 keeps everything together, i.e., all the group elements 4 are held by the super structure 5 via mounting elements 17 for evenly distributing the diode patches 2 along a double curved surface adapted to the head of a user, as described hereinafter.

According to the embodiment shown in the figures, each diode patch 2 positioned in the innermost structural level is essentially hexagonal shaped and the diode systems 3 are essentially located on the corner points of a hexagon. Each diode system 3 comprises a diode 3a, e.g., a laser diode LD (or an injection laser diode (ILD) or semiconductor laser or diode laser) or a light-emitting diode LED, i.e., a semiconductor device, configured to emit a beam of laser light or LED-light, respectively, having a wavelength of around 650nm or between 800nm and 1100nm, depending on the therapeutic goal, i.e., a therapeutical treatment of androgenetic alopecia or therapeutic stimulation of the brain via transcranial laser therapy (TLT).

In addition, the lens system 3b also defining the optical axis O is focusing the emitted light beam to a narrow bundle positioned on said optical axis O increasing the light intensity at the target location, e.g. where the light engages the scalp of a user. Thereby, a higher light intensity increases the penetrative power of the emitted light through the scalp, for example, and into the brain tissue enabling treatment at a higher depth, e.g., in case of therapeutic stimulation of the brain via transcranial laser therapy (TLT). In case of therapeutical treatment of androgenetic alopecia higher light intensity increases the effect of the red light (around 650nm) on the hair follicles in the scalp of the user, for example.

Thereby, the lens system 3b ensures that the emitted laser light or LED light converges along the optical axis O to concentrate the projected light optical output power on a small surface of the scalp. The focal distance of the lens system 3b is about 20mm from the rounded tip 2d of the finger-shaped part 2c2 (as described later) or the surface 2g of the flat part 2c1 (as described later) to ensure safety by avoiding potential pinpoint accumulation of light energy that can potentially cause burning of the skin of the scalp.

Further, at least some of the diode patches 2, preferably, two of the most frontal oriented diode patches 2 in a front area 1F of the laser therapy head covering 1a, as shown in more detail in Figs. 7 and 8, comprises a (double) proximity sensor system 3c that can measure the distance to the surface in its detection area. Once it is determined using the proximity sensor system 3c that the distance between a surface of an object and the respective proximity sensor system 3c is too large, the total light therapy head covering 1a, i.e., all the diode patches 2, is electronically stopped from emitting light. Thereby, the proximity sensor systems 3c are placed in such a way that if not placed safe, e.g., the distance of the nearest object is larger than a predefined distance-threshold, specifically in case the two most frontal oriented diode patches 2 are positioned above the eyes, the emission of light is stopped. This is a safety feature to safeguard against damage to eyes due to improper placement of the light therapy head covering 1a and to only enable the light therapy head covering 1a to emit light when properly placed on the head. The use of two proximity sensor systems 3c is a redundancy in case the user covers one proximity sensor system 3c while handling the light therapy head covering 1a.

Each diode system 3 is located inside a front housing part 2a of the diode patch 2 and is connected to a patch circuit board 2b, e.g., a printed circuit board, wherein the diode 3a of the respective diode system 3 might be soldered to the corresponding conducting paths on the patch circuit board 2b. In this way, the diode systems 3 of the diode patch 2 can be individually controlled, e.g., by an electronic control unit ECU centrally located on the light therapy head covering 1a, as described herein later. Thereby, the hexagonal shape of the preferred embodiment of the invention allows for six flat sides of the patch circuit board 2b between the corner points of the hexagon. This is optimal for the placement of a connector C1 for attaching a cable C2 to the patch circuit board 2b, as shown in the figures, allowing to electronically interconnect different diode patches 2 and/or providing an electronic connection to the central electronic control unit ECU, without much effort.

Each diode system 3 is surrounded and protected by a housing, wherein the exact construction of the housing is dependent on the location of the respective diode patch 2 on the light therapy head covering 1a. Preferably, diode patches 2 that are located in the front part 1F of the light therapy head covering 1a comprise a scalp facing housing part 2c that is formed as a flat part 2c1 having an essentially flat or essentially planar surface 2g, if at all, with only a slight curvature, as shown in 7 in more detail. On the other hand, diode patches 2 that are located in a left part 1L, a right part 1R, an upper part 1U and a rear part 1B of the light therapy head covering 1a comprise a scalp facing housing part 2c that includes several spaced-apart finger-shaped parts 2c2, each assigned to a single diode system 3 of the respective diode patch 2, as shown in Fig. 3, for example.

Furthermore, the flat or planar surface 2g is covered with a soft material such as silicone rubber. This enables the user to place the diode patches 2 that are located in the front part 1F of the light therapy head covering 1a on the forehead comfortably and enable the user to carefully position these diode patches 2 to ensure that the light therapy head covering 1a is placed correctly with the eyes not be exposed to laser light. As described above, a safety feature enabled by a double proximity sensor system 3c, ensures that the diode patches 2 do not emit light when not placed correctly.

Thereby, the surface 2g of the flat part 2c1 is perpendicular to the optical axis O of the respective diode system 3 and thus oriented to the scalp of the user, once the light therapy head covering 1a is placed on the head of the user. The surface 2g of the flat part 2c1 might also be made of a soft material such as a silicone rubber to provide a comfortable pressure area for the user's head, as the surface 2g of the flat part 2c1 is located on the diode patches 2 in the front part 1F of the light therapy head covering 1a that are placed or pressed onto the forehead of a user, in particular during adjusting the light therapy head covering 1a, as described herein later.

Further, each finger-shaped part 2c2 of the respective diode patch 2 is arranged in such a way that it acts as a kind of light finger that is oriented along the direction of the optical axis O and directed to the scalp of the user, once the light therapy head covering 1a is placed on the head of the user. Preferably, the finger-shaped part 2c2 is integrated into the front housing part 2a of the diode patch 2 in such a way that a compact construction with a smooth transition is achieved which facilitates cleaning. In addition, the finger-shaped part 2c2 of the front housing part 2a forms a hollow light guide along the optical axis O having a nearly cylindrical or frustoconical shape that transitions into a rounded tip 2d at the distal end providing more user comfort. In particular, the respective finger-shaped part 2c2 with the rounded tip 2d can push aside strands of hair in a similar way as the rounded teeth of a comb. This results in the rounded tip 2d touching the scalp or being located close to the scalp so that the user's hair does not block the emitted light that ideally exits the finger-shaped part 2c2 at the rounded tip 2d or that the blocking of the emitted light by the user's hair is at least significantly reduced.

Further, the diode systems 3 and the finger-shaped parts 2c2 surrounding them are positioned on the diode patches 2 in such a way that each of the rounded tips 2d can touch the convex shape of the scalp in case the respective diode patch 2 is more or less tangent or parallel to the curvature of this convex shape of the user's scalp. In other words, each diode system 3, each optical axis O and each finger-shaped part 2c2 that is assigned to a certain diode patch 2 is vertically aligned to a (virtual) curved plane P that has a convex shape of a (standard) user's scalp, as indicated in Fig. 5, i.e., a curved plane P.

Using such an arrangement of diode systems 3 and scalp facing housing parts 2c; 2c1, 2c2 within a diode patch 2 makes the incidence of the diode systems 3 and the optical axis O more or less perpendicular to the scalp of the user also optimizing the incidence of the beam of emitted laser light or LED-light and, thus, optimizing the impact of the emitted light onto the scalp in case of therapeutical treatment of androgenetic alopecia, or optimizing the penetration of the emitted light through the boney tissue and into the brain tissue in case of therapeutic stimulation of the brain (TLT).

Furthermore, a length L2c (or thickness) of the scalp facing housing part 2c, i.e., the flat part 2c1 or the finger-shaped part 2c2, (measured parallel to the direction of the light propagation or the optical axis O) and the construction of the diode patches 2 promote that heat that is generated by the diode 3a is kept away from the scalp of the user which keeps the head of the user relatively cool and avoids the problem of discomfort and hence drop in compliance. Therefore, a length L2c of at least 10mm is chosen.

Preferably, the diode patches 2 contain a patch circuit board 2b with a back side 2e (opposite to the conducting paths) made of a heat conductive material, like the aluminum alloy 1050A, as also indicated in Fig. 3. In a preferred embodiment the back side 2e of the patch circuit board 2b is covered by an additional back housing part 2f forming the back side of the diode patch 2 wherein the back housing part 2f is also made of an aluminum alloy for dissipating heat. The back housing part 2f is attached to the front housing part 2a of the respective diode patch 2.

As this heat conductive material is located on the back side 2e of the patch circuit board 2b and the back housing part 2f of the diode patch 2, each facing away from the scalp or the rounded tips 2d being in close distance to the scalp or the surface 2g of the flat part 2c1 being in close distance to the scalp, this construction enables the dissipation of the heat that is generated in the process of the generation of light by the diodes 3a. As the heat conductive material is facing away from the scalp of the user, the heat is dissipated away from the head of the user, providing a secure solution. Furthermore, because the diode patches 2 form an open structure due to the spaced apart finger-shaped parts 2c2 within the respective diode patch 2 in the left part 1L, the right part 1R, the upper part 1U and the rear part 1B of the light therapy head covering 1a as well as the distance between neighboring diode patches 2, any heat that still dissipates towards the scalp can easily escape with air circulating freely between the diode patches 2 and the finger-shaped parts 2c2 (where applicable).

On the second structural level, group elements 4 are formed by interconnecting a certain number of functional modules **F,** i.e., diode patches 2 in the shown embodiment, via a springy supporting element 7. Preferably, each supporting element 7 is made of a polypropylene material, for example, allowing for relatively easy optimization in preproduction of the springy behavior or flexibility and the shape to provide sufficient support for the respective diode patches 2 in different directions, if necessary. Each supporting element 7 comprises a central portion 8 from which several springy arms 9 are protruding spider-like in different directions, wherein the number of springy arms 9 is determined in dependance of the number of diode patches 2 assigned to the respective group element 4. Further, the dimensions of these springy arms 9 and the arrangement within the respective group element 4 is specifying the shape and the size of the respective group element 4.

Thereby, different group elements 4 evenly distributed over the area of the light therapy head covering 1a may have different sizes, different numbers of diode patches 2 and different arrangements of the diode patches 2. Further, the diode patches 2 interconnected within these group elements 4 can be combined or pooled in patch arrangements 6 of various shapes and sizes. In a preferred embodiment of the invention, there are in total five different patch arrangements 6 distributed over the whole light therapy head covering 1a, as best seen in Fig. 2, wherein each patch arrangement 6 contains at least one group element 4. These five different patch arrangements 6 represent the five different areas of the brain that are typically distinguished anatomically as they represent different brain functions. These areas are
- the frontal area represented by a front patch arrangement 6F located in the front part 1F of the light therapy head covering 1a combining in total seven diode patches 2 within two group elements 4 (see Figs. 2, 7),
- the temporal right area represented by a right patch arrangement 6R located in the right part 1R of the light therapy head covering 1a combining in total five diode patches 2 within one group element 4 (see Figs. 2, 4, 5, 6),
- the temporal left area represented by a left patch arrangement 6L located in the left part 1L of the light therapy head covering 1a combining in total five diode patches 2 within one group element 4 (see Figs. 2, 4, 5, 6),
- the parietal area represented by an upper patch arrangement 6U located in the upper part 1U of the light therapy head covering 1a combining in total fourteen diode patches 2 within four group elements 4 (see Fig. 2), and
- the occipital area represented by a rear patch arrangement 6B located in the rear part 1B of the light therapy head covering 1a combining in total seven diode patches 2 within two group elements 4 (similar to the frontal area but with finger-shaped parts 2c2 instead of the flat part 2c1). As these areas represent anatomical areas with different functionalities, this forms an easy to understand and thus operate system.

For attaching the respective number of diode patches 2 within a group element 4 via the supporting element 7, a distal end 9a of the respective springy arm 9 is attached to a central connecting area 10 of a diode patch 2 via a fastener 11. Preferably, the fastener 11 also ensures that the distal end 9a of the respective springy arm 9 is not directly touching the back housing part 2f of the diode patch 2, thus leaving a certain distance between both elements. Therefore, the fastener 11 comprises a cap extender 11a, as shown in Fig. 3, that is attached to the back housing part 2f of the respective diode patch 2, thus acting as a spacer. The distal end 9a of the respective springy arm 9 can be attached to this cap extender 11a via an end cap 11b of the fastener 11, e.g., having a screw thread allowing to receive a screw 11c of the fastener 11 for securely mounting the diode patch 2, or via any other attachment means. A further diode patch 2 may be directly attached to the central portion 8 of the supporting element 7 via said fastener 11, exemplary shown in Fig. 4, meaning that the number of springy arms 9 of the supporting element 7 equals the number of diode patches 2 per group element 4 minus one.

On the third level is the super structure 5 intended to adjust and fasten the light therapy head covering 1a to the head of a user. Thereby, the super structure 5 of the light therapy head covering 1a is where the user holds the light therapy head covering 1a while positioning it on the head, consequently, the super structure 5 has to be robust and easy to handle. In a preferred embodiment of the invention, also shown in more detail in Figs. 8 to 11, the super structure 5 comprises three semicircular rigid elastic elements 12, e.g., containing or made of blade springs B (two blade springs B in the shown example), assembled in a three-dimensional or spherical configuration defining an interior space S for accommodating the head of a user. A front rigid elastic element 12a extending over the front part 1F is intended to be positioned on the front of the head of the user, a rear rigid elastic element 12b extending over the rear part 1B is intended to be located on the back of the head of the user and an upper rigid elastic element 12c extending over the upper part 1U is intended to bridge the upper head of the user. Thereby, semi-circular rigid elastic element 12 is to be understood as an element that in its essentially semi-circular form is stiff and has the flexibility and elasticity constrained to a single plane, wherein it can bend under external forces, store potential energy, and return to their original shape (neutral position) upon the removal of those forces. This can be accomplished by said blade spring B, for example, with a neutral position or free state in a semi-circular shape.

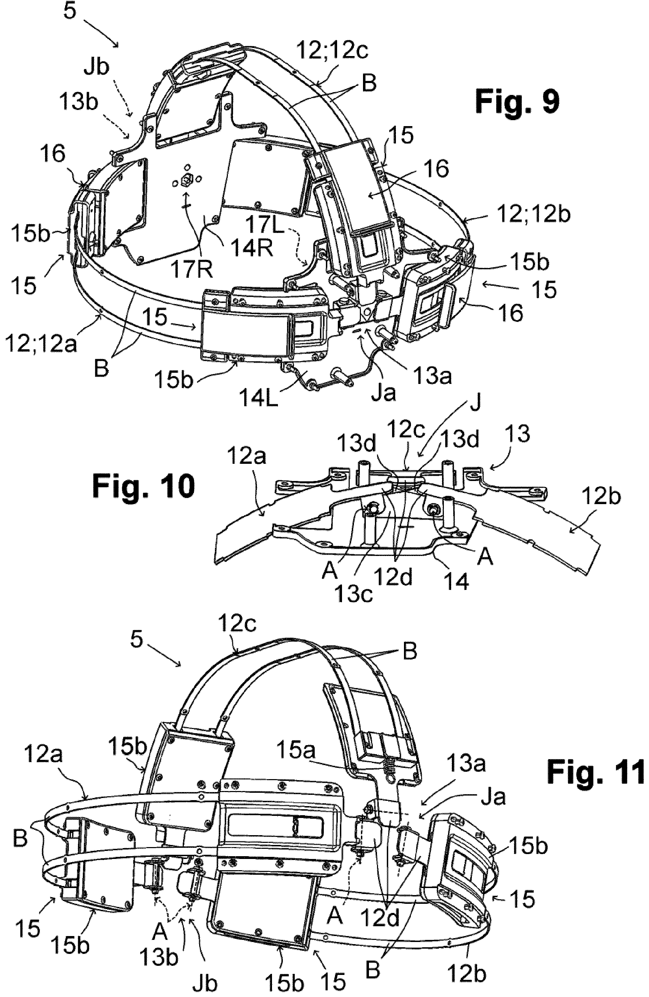

Further, the rigid elastic elements 12 are joined together in joining areas J, i.e., a left joining area Ja on the left part 1L of the medical head covering 1 and a right joining area Jb on the right part 1R of the medical head covering 1a, so as to partially enclose said interior space S. Thereby, as shown in more detail in Fig. 9 and 10, the left joining area Ja and the right joining area Jb is formed by hinge connections 13, i.e., a left hinge connection 13a and a right hinge connection 13b, each receiving one end of the three rigid elastic elements 12; 12a, 12b, 12c in a movable manner such that each of the three rigid elastic elements 12; 12a, 12b, 12c run between both hinge connections 13; 13a, 13b. Consequently, a rigid connection of the rigid elastic elements 12; 12a, 12b, 12c when they are joined together at the hinge connections 13; 13a, 13b is omitted. These hinge connections 13; 13a, 13b on the sides of the super structure 5 enable the three-dimensional spherical structure of the light therapy head covering 1a to bend in lateral direction to accommodate for different shapes of the heads of users in the interior space S.

Each hinge connection 13; 13a, 13b is integrated into a main body element 14 of the light therapy head covering 1a, i.e., a left main body element 14L and a right main body element 14L, each being positioned near to an ear of the user, for example, as this is the widest point of the human skull and where the curvature of the skull is tangent with the vertical axis. Each movably fixed rigid elastic element 12; 12a, 12b, 12c protrudes through an opening 14a, 14b, 14c in the respective main body element 14; 14L, 14R, i.e., a front opening 14a, a back opening 14b, a top opening 14c, respectively, and from there the respective rigid elastic element 12; 12a, 12b, 12c runs to the hinge connection 13; 13a, 13b on the other side of the light therapy head covering 1a in a semicircular manner, i.e., semicircular in the front area 1F, the rear area 1B and the upper area 1U of the light therapy head covering 1a, respectively.

Moreover, each hinge connection 13 provides a limited range of movement of the accommodated rigid elastic elements 12; 12a, 12b, 12c that is carefully determined so that the structure of the light therapy head covering 1a in the side areas 1L, 1R of the hinge connections 13; 13a, 13b follows the shape of a head circumference as good as possible in all positions, from totally closed to maximally opened. In a preferred embodiment of the invention, that is shown in detail in Fig. 10, the limitation in movement is provided by finger-shaped ends 12d of the respective rigid elastic elements 12 that are each pivot-mounted around a pivot axis A on a protrusion 13c integrally formed within the respective main body element 14; 14L, 14R. Further, each finger-shaped end 12d rests on an inclined surface 13d that is formed in the protrusion 13c. In this way, the pivotal movement around the pivot axis A is limited.

Further, each of these rigid elastic elements 12; 12a, 12b, 12c contain two sliding mechanisms 15, one on each end of each rigid elastic element 12; 12a, 12b, 12c, allowing to individually adjust the lengths of the respective rigid elastic element 12; 12a, 12b, 12c as they are slidingly extendable and retractable. In this way, the super structure 5 can be adjusted between an open position in which the diameter of the light therapy head covering 1a or the volume of the interior space S is maximal, and a use position in which the diameter of the light therapy head covering 1a or the volume of the interior space S is reduced such that the light therapy head covering 1a fits to the head of the respective user. Thereby, each rigid elastic element 12; 12a, 12b, 12c can be adjusted individually for flexible adaptation of the interior space S to the shape and size of the head of the user.

Preferably, these sliding mechanisms 15 have a pulling spring 15a (see Fig. 10) that keeps the sliding mechanism 15 preloaded into a fully closed position, in which the diameter of the light therapy head covering 1a or the volume of the interior space S is minimal. Thereby, the pulling spring 15a of the sliding mechanism 15 is fine tuned to pull enough but not too much. Consequently, after the light therapy head covering 1a is put on the user's head, the length of the respective rigid elastic element 12; 12a, 12b, 12c is held by the pulling spring 15a and friction in the system, resulting in a high enough "clamping" force but omitting an uncomfortable seat for the user. However, the pulling spring 15a can also be omitted and the closed position or use position can be set otherwise.

Further, the blade springs B of the rigid elastic elements 12; 12a, 12b, 12c are movably guided in a guiding part 15b of the respective sliding mechanisms 15. Thereby, the finger-shaped ends 12d received by the hinge connections 13, are connected to these guiding parts 15b such that when a (manual) pulling force is applied against the pulling spring 15a, e.g. via a lever 16, the rigid elastic elements 12; 12a, 12b, 12c can precisely and reproducibly slide open or into the open position extending the length of the respective rigid elastic elements 12; 12a, 12b, 12c in a comfortable way and, therefore, increasing the diameter of the light therapy head covering 1a or the volume of the interior space S allowing to comfortably stretch the shape of the super structure 5 to fit to different head shapes or head sizes (horizontal and transversal).

After the user has placed the light therapy head covering 1a on his head and adjusted the size and shape accordingly, the respective rigid elastic elements 12; 12a, 12b, 12c of the super structure 5 are locked or held in place solely by the pulling spring 15a and/or by an additional locking mechanism (not shown), preventing the rigid elastic elements 12; 12a, 12b, 12c to be extended or retracted. Applying the lever 16, a latch, a button, or any other means acting in a similar way, allows for further extension or retraction of the rigid elastic elements 12; 12a, 12b, 12c, i.e., against the pulling spring 15a and/or a locking mechanism (if applicable), e.g., in case removing or readjusting the light therapy head covering 1a is intended. The extension and/or retraction of the rigid elastic elements 12; 12a, 12b, 12c and/or the locking and/or releasing may be performed manually by the user or automatically via electromotors or a combination thereof.

Summarizing, a three-dimensional semi-rigid super structure 5 is provided that can fit different head sizes as well as different head shapes, ensuring an optimized fit of the medical head covering 1a or light therapy head covering 1a. Thereby, the totality of the three-dimensional spherical structure of the rigid elastic elements 12; 12a, 12b, 12c, the sliding elements 15 and the joining areas J; Ja, Jb, especially formed as hinge connections 13; 13a, 13b, provides that the super structure 5 has the flexibility to follow the shape and sizes of a large variety of heads while also providing the rigidity of a framework that is necessary to attach the group elements 4 in their respective positions and with their respective weights in a robust manner.

Further, the group elements 4 described above are mounted to the super structure 5 via various mounting elements 17, wherein the group elements 4 within the front patch arrangement 6F, the rear patch arrangement 6B and the upper patch arrangement 6U are attached to the respective rigid elastic element 12; 12a, 12b, 12c, in particular, to the blade springs B of the respective rigid elastic element 12; 12a, 12b, 12c, and the group elements 4 within the left patch arrangement 6L and the right patch arrangement 6R are attached to the left and right main body element 14L,14R, respectively.

Thereby, the connection between the group elements 4 and the main body elements 14L,14R is rigid, provided that the supporting elements 7 already provide enough flexibility. Therefore, a left mounting element 17L is integrated in the left main body element 14L and a right mounting element 17R is integrated in the right main body element 14L, as indicated in Fig. 9. The left and right mounting elements 17L, 17R may comprise a screw (or a nut) or the like allowing to rigidly attach the supporting element 7 (e.g., via a counter mounting element 17d (nut or screw) on the central portion 8) of the left patch arrangement 6L and the right patch arrangement 6R, respectively, to the respective main body element 14L, 14R via a screwed connection (nutscrew-connection), for example.

In addition, the connections between the group elements 4 and the rigid elastic elements 12; 12a, 12b, 12c are rigid in some places and allow for a certain angular adjustment in other places, depending on the required fit of the functional modules F or diode patches 2 to the scalp of the user. As the rigid elastic elements 12; 12a, 12b, 12c and supporting elements 7 are flexible themselves, the connection can be more rigid in some places. For example, the diode patches 2 in the upper patch arrangement 6U can be more rigid, wherein group elements 4 combined within the upper patch arrangement 6U are interconnected by a rigid top frame element 17a (see Fig. 1 and 2) that is attached to the central portions 8 of the respective supporting elements 7, e.g., via a screwed connection. The top frame element 17a, in turn, is rigidly connected to an upper mounting element 17U which is clamped to the upper rigid elastic element 12c, e.g., the blade springs B thereof. The upper mounting element 17U may comprise a screw (or a nut) or any clipping means or the like allowing to rigidly attach the top frame element 17a of the upper patch arrangement 6U to the upper rigid elastic element 12c. Thereby, the flexibility of the supporting elements 7 and the upper rigid elastic element 12c is sufficient and the rigid interconnection ensures that the light therapy head covering 1a has a structural robustness that allows for ease of handling.

However, to ensure a good fit of the diode patches 2 with the scalp, the connection in some places, e.g., the front and the back, has to allow for a larger angular adjustment. Therefore, in a preferred embodiment of the invention, a front mounting element 17F and a read mounting element 17B clamped to the front rigid elastic element 12a, e.g., the blade springs B thereof, and the rear rigid elastic element 12b, e.g., the blade springs B thereof, respectively, additionally comprise a cup structure 17b allowing to movably accommodate a ball structure 17c rigidly attached to the supporting structure 7 of the group elements 4 combined within the front patch arrangement 6F and the rear patch arrangement 6B, respectively, as shown in Fig. 2, 7 and 8. Thereby, ball joints are formed allowing and ensuring angular adjustment with angular limitation of the group elements 4 within the front patch arrangement 6F and the rear patch arrangement 6B against the front and rear rigid elastic elements 12a, 12b for correct positioning of the group elements 4 relative to the front and rear rigid elastic elements 12a, 12b that they are connected to. In addition, two further ball joints are located near the sliding mechanisms 15 of each of the respective rigid elastic elements 12a, 12b. On the support structures 7 on the front and back, these ball joints are located on the distal ends 9a of one of the springy arms 9, for example, as shown in Figs. 2 or 8. This ensures optimal special flexibility while at the same time providing a robust structure element to the light therapy head covering 1a that is easy to use for the user.

In a preferred embodiment of the invention, in preparing for the positioning of the light therapy head covering 1a on the head of the user, the lever 16 or similar means assigned to the rear rigid elastic elements 12b of the super structure 5 are pulled or pushed to unlock or release the rear rigid elastic element 12b and prepare for the opening solely of the rear rigid elastic element 12b. In this half-opened position, the front rigid elastic element 12a of the super structure 5 comprising the front patch arrangement 6F with diode patches 2 comprising the scalp facing housing part 2c that is formed as a flat part 2c1 having an essentially flat or essentially planar surface 2g, in particular, having a slight curvature, can be placed on the forehead of the user and the user can now pull the super structure 5 backwards.

As the flat part 2c1 is made of a soft rubber material even applying a high force during the placement and adjustment of the light therapy head covering 1a is comfortable. Thereby, the user is pulling the light therapy head covering 1a backwards holding it on the main body elements 14; 14L, 14R on the right and left side which automatically opens or releases or unlocks the sliding mechanism 15 on the front rigid elastic element 12a, e.g., due to a high force (or it is unlocked otherwise). This allows to adjust the length of the front rigid elastic element 12a via the respective sliding mechanism 15. A similar procedure can be used to adjust the lengths of the upper rigid elastic element 12c.

In this way, the entire super structure 5 or the volume of the interior space S can be adjusted by repositioning or readjusting the different structural parts, i.e., the rigid elastic elements 12; 12a, 12b, 12c, of the light therapy head covering 1a. Thereby, the user adjusts the position and sizing of the light therapy head covering 1a by manipulating or pushing and pulling the main body elements 14; 14L ,14R on the right and left side until the light therapy head covering 1a fits snug and comfortable.

Further, the springy arms 9 of the second structural level group elements 4 enable the repositioning of the diode patches 2 comprising the respective scalp facing housing part 2c of the front housing part 2a guiding the light to the scalp of the user. The repositioning of the light therapy head covering 1a ensures that the finger-shaped parts 2c2 within the left, right, upper and rear patch arrangements 6L, 6R, 6U, 6B can push through the hair and find their position close to the surface of the scalp so that the light generated by the diodes 3a is not blocked by the hair, optimizing the respective therapeutical effect. While the positioning of the finger-shaped parts 2c2 relative to the scalp will sometimes be a little off, hair grows at an angle to the scalp so that the light beams can still reach the scalp in that case, i.e., still ensuring an effective therapeutical effect.

In the preferred embodiment of the invention, a centralized main circuit board 20, e.g., a printed circuit board, is located inside one of the main body elements 14; 14L, 14R, e.g., the left main body element 14L, of the light therapy head covering 1a. The electronic control unit ECU is electronically connected, e.g., soldered, to this main circuit board 20. Via the connectors C1 and the cables C2 and further centralized cables, the different patch circuit boards 20 are electronically connected to the main circuit board 20 through an intricate system of cables. To allow for positioning of these cables through the structures of the light therapy head covering 1a that are flexible and that can change their lengths, e.g., via the described sliding mechanisms 15, these cables are configured in a ribbon style or a flexible printed cable (FPC) style. In a preferred embodiment of the invention these cables are overmoulded with a flexible and protective rubberlike material for protection.

This cabling allows the electronic control unit ECU to individually address each diode 3a in the several diode patches 2 in a centralized way to allow specific treatments of selected parts of the brain individually. Other embodiments may have all diodes 3a addressed and controlled together.

In a preferred embodiment of the invention, the electronic control unit ECU is configured to be wirelessly controlled by an external device 30, such as a smartphone, tablet or personal computer, e.g., via a Bluetooth connection. Alternatively, the electronic control unit ECU can be controlled by an external controller via a wired connection.

In the embodiments described above, diode patches 2 as functional modules F of this light therapy head covering 1a are described. However, using electrodes as functional modules F, e.g., for electroencephalography (EGG), used for diagnostic purposes can be used in conjunction with the super structure 5 described above, providing the same benefits concerning a structural robustness that allows for ease of handling for easy and secure fitting of the electrodes to the scalp of a user.

### List of reference signs

- 1: medical head covering
- 1a: light therapy head covering
- 1B: rear part of the medical head covering 1a
- 1F: front part of the medical head covering 1a
- 1L: left part of the medical head covering 1a
- 1R: right part of the medical head covering 1a
- 1U: upper part of the medical head covering 1a
- 2: diode patch
- 2a: front housing part
- 2b: patch circuit board
- 2c: scalp facing housing part of the front housing part 2a
- 2c1: flat part of the front housing part 2a
- 2c2: finger-shaped part of the front housing part 2a
- 2d: rounded tip of the finger-shaped part 2c2
- 2e: back side of the circuit board 2b
- 2f: back housing part
- 2g: surface of the flat part 2c1
- 3: diode system
- 3a: diode
- 3b: lens system
- 3c: proximity sensor system
- 4: group element
- 5: super structure
- 6: patch arrangement
- 6B: rear patch arrangement
- 6F: front patch arrangement
- 6L: left patch arrangement
- 6R: right patch arrangement
- 6U: upper patch arrangement
- 7: supporting element
- 8: central portion of the supporting element 7
- 9: springy arms
- 9a: distal end of the springy arm 9
- 10: connecting area of the diode patch 2
- 11: fastener
- 11a: cap extender
- 11b: end cap
- 11c: screw
- 12: rigid elastic element
- 12a: front rigid elastic element
- 12b: rear rigid elastic element
- 12c: upper rigid elastic element
- 12d: finger-shaped end of the rigid elastic element 12
- 13: hinge connection
- 13a: left hinge connection
- 13b: right hinge connection
- 13c: protrusion
- 13d: inclined surface
- 14: main body element
- 14a: front opening
- 14b: back opening
- 14c: top opening
- 14L: left main body element
- 14R: right main body element
- 15: sliding mechanism
- 15a: pulling spring
- 15b: guiding part
- 16: lever
- 17: mounting element
- 17B: rear mounting element
- 17F: front mounting element
- 17L: left mounting element
- 17R: right mounting element
- 17U: upper mounting element
- 17a: top frame element
- 17b: cup structure
- 17c: ball structure
- 17d: counter mounting element
- 20: main circuit board
- 30: external device
- A: pivot axis
- B: blade spring
- C1: connector
- C2: cable
- ECU: electronic control unit
- F: functional modules
- J: joining area
- Ja: left joining area
- Jb: right joining area
- L2c: length of the scalp facing housing part 2c
- O: optical axis
- P: curved plane
- S: interior space

## Claims

1. A medical head covering (1) for the treatment and/or diagnosis of a user's head, comprising:
- functional modules (F) for the treatment and/or diagnosis of the user's head; and
- a super structure (5) comprising at least three essentially semicircular rigid elastic elements (12; 12a, 12b, 12c) joined together in a joining area (J) so as to partially enclose an interior space (S) having a curved surface for accommodating the head of the user, wherein
--- at least a front rigid elastic element (12a) that is extending in a front part (1F) of the medical head covering (1) and at least one rear rigid elastic element (12b) that is extending in a rear part (1B) of the medical head covering (1) are joined together in the joining area (J) so as to form a rigid and elastic circle or ellipse around the interior space (S), and
--- at least one upper rigid elastic element (12c) is extending in an upper part (1U) of the medical head covering (1) and partly enclosing the interior space (S) from above,
whereby the functional modules (F) are connected to the super structure (5) individually or in groups via mounting elements (17), either rigidly or movably, in such a way that they are oriented into the interior space (S) for approaching or touching the user's head,
**characterized in that**
the rigid elastic elements (12; 12a, 12b, 12c) are each joined together in a left joining area (Ja) in a left part (1L) of the medial head covering (1) and in a right joining area (Jb) in a right part (1R) of the medical head covering (1), respectively, wherein the left part (1L) and the right part (1R) are located opposite to each other within the medical head covering (1),
wherein the rigid elastic elements (12; 12a, 12b, 12c) each comprise a sliding mechanism (15) for individually adjusting the length of the respective rigid elastic element (12; 12a, 12b, 12c) for flexible adaptation of the interior space (S) to the shape and size of the head of the user, and
wherein the rigid elastic elements (12; 12a, 12b, 12c) are joined together in the respective joining area (J; Ja, Jb) via hinge connections (13; 13a, 13b), wherein each hinge connection (13; 13a, 13b) receives one end of the at least three rigid elastic elements (12; 12a, 12b, 12c) such that each of the at least three rigid elastic elements (12; 12a, 12b, 12c) can pivot against each other in the respective joining area (J; Ja, Jb) to allow for adaptation to different shapes of the users head.

2. The medical head covering (1) according to claim 1, wherein the rigid elastic elements (12; 12a, 12b, 12c) each comprise or are made of at least one blade spring (B) with a neutral position or free state in a semi-circular shape, wherein the at least one blade spring (B) is preferably made of metal.

3. The medical head covering (1) according to claim 2, wherein the respective sliding mechanism (15) comprises a guiding part (15b), wherein the at least one blade spring (B) is at least partially, e.g., with its end section, movably accommodated and guided by said guiding part (15b) such that the at least one blade spring (B) can slidingly move against the respective guiding part (15b) for allowing to slidingly extend and retract the respective rigid elastic element (12; 12a, 12b, 12c).

4. The medical head covering (1) according to claim 3, wherein the respective sliding mechanism (15) comprises a pulling spring (15a) connecting the at least one blade spring (B) and the respective guiding part (15b) in a springy manner preloading the respective rigid elastic element (12; 12a, 12b, 12c) into a retracted position.

5. The medical head covering (1) according to any of the preceding claims, wherein each of the at least three rigid elastic elements (12; 12a, 12b, 12c) can pivot around a pivot axis (A) extending perpendicular to the semi-circular propagation direction of the respective rigid elastic element (12; 12a, 12b, 12c) to allow for adaptation to different shapes of the users head.

6. The medical head covering (1) according to any of the preceding claims, wherein each of the hinge connections (13; 13a, 13b) comprises a protrusion (13c) having an inclined surface (13d), wherein the respective rigid elastic element (12; 12a, 12b, 12c) comprises a finger-shaped end (12d) pivot-mounted around the pivot axis (A) on the protrusion (13c) such that the finger-shaped end (12d) of the respective rigid elastic element (12; 12a, 12b, 12c) rests on the inclined surface (13d) for limiting the rotational movement of the respective rigid elastic element (12; 12a, 12b, 12c) around the pivot axis (A).

7. The medical head covering (1) according to any of the preceding claims, wherein the functional modules (F) are diode patches (2), each having at least three, preferably six, diode systems (3) with diodes (3a) emitting light along an optical axis (O) that is defined by a lens system (3b) of the respective diode system (3), wherein the diodes (3a) of the diode systems (3) of the respective diode patch (2) are preferably configured to emit laser light or LED-light having a wavelength of approx.. 650nm for therapeutical treatment of androgenetic alopecia or between 800nm and 1100nm for the therapeutic stimulation of the brain via transcranial laser therapy, for providing a light therapy head covering (1a) as medical head covering (1).

8. The medical head covering (1) according to claim 7, wherein the diode systems (3) of the diode patches (2) are surrounded by a front housing part (2a) of the respective diode patch (2), wherein said front housing part (2a) of at least one of the diode patches (2) is formed by
- at least three, preferably six, transparent finger-shaped parts (2c2) spaced apart from each other, wherein each transparent finger-shaped part (2c2) is assigned to one of the diode systems (3) of the respective diode patch (2) and each finger-shaped part (2c2) is oriented in the direction of the optical axis (O) of the assigned diode system (3) such that the emitted light propagates though the finger-shaped part (2c2) and exits the respective finger-shaped part (2c2) at its distal end formed as a rounded tip (2d); or
- a flat part (2c1) surrounding all the diode systems (3) of the respective diode patch (2) and having a flat surface (2g), in particular a curved flat surface (2g).

9. The medical head covering (1) according to claim 7 or 8, wherein the diode patches (2) further comprise a back housing part (2f) forming the backside of the respective diode patch (2), wherein the back housing part (2f) contains or is made of a heat conductive material, e.g., aluminum alloy 1050A.

10. The medical head covering (1) according to any of the preceding claims, wherein the functional modules (F) are springy combined within group elements (4), wherein each group element (4) comprises at least three of said functional modules (F) that are spaced apart from each other and that are springy connected to each other via a supporting element (7), wherein the supporting element (7) has at least three springy arms (9) protruding in different directions, wherein each springy arm (9) is connected to one of the at least three functional modules (F) for springy carrying the respective functional module (F).

11. The medical head covering (1) according to any of the preceding claims, wherein at least one of the mounting elements (17) connecting the respective functional module (F) to the super structure (5), in particular to the rigid elastic element (12; 12a, 12b, 12c) of the super structure (5), comprises a cup structure (17b) allowing the movable reception of a ball structure (17a) to which the functional module (F) is attached individually or within a group for allowing an angular adjustment of the respective functional module (F) against the super structure (5).

12. A medical head covering (1) according to any of the preceding claims, wherein it further comprises an electronic control unit (ECU) electronically connected to the functional modules (F) for controlling treatment and/or diagnoses of the user's head via these functional modules (F), wherein the electronic control unit (ECU) is located in a main body element (14) of the medical head covering (1), in particular, surrounding one of the joining areas (J), comprising a main circuit board (20), electronically connected to the several functional modules (F) via cables (C2).

## Patentansprüche

1. Medizinische Kopfbedeckung (1) zur Behandlung und/oder Diagnose des Kopfes eines Benutzers, umfassend:
- Funktionsmodule (F) zur Behandlung und/oder Diagnose des Kopfes des Benutzers; und
- eine Superstruktur (5), die mindestens drei im Wesentlichen halbkreisförmige starre elastische Elemente (12; 12a, 12b, 12c) umfasst, die in einem Verbindungsbereich (J) derart miteinander verbunden sind, dass sie einen Innenraum (S) mit einer gekrümmten Oberfläche zur Aufnahme des Kopfes des Benutzers teilweise umschließen, wobei
--- mindestens ein vorderes starres elastisches Element (12a), das sich in einem vorderen Teil (1F) der medizinischen Kopfbedeckung (1) erstreckt, und mindestens ein hinteres starres elastisches Element (12b), das sich in einem hinteren Teil (1B) der medizinischen Kopfbedeckung (1) erstreckt, in dem Verbindungsbereich (J) derart miteinander verbunden sind, dass sie einen starren und elastischen Kreis oder eine Ellipse um den Innenraum (S) bilden, und
--- mindestens ein oberes starres elastisches Element (12c) sich in einem oberen Teil (1U) der medizinischen Kopfbedeckung (1) erstreckt und den Innenraum (S) von oben teilweise umschließt,
wobei die Funktionsmodule (F) einzeln oder in Gruppen über Befestigungselemente (17) entweder starr oder beweglich derart mit der Superstruktur (5) verbunden sind, dass sie in den Innenraum (S) ausgerichtet sind, um sich dem Kopf des Benutzers anzunähern oder diesen zu berühren,
**dadurch gekennzeichnet, dass**
die starren elastischen Elemente (12; 12a, 12b, 12c) jeweils in einem linken Verbindungsbereich (Ja) in einem linken Teil (1L) der medizinischen Kopfbedeckung (1) beziehungsweise in einem rechten Verbindungsbereich (Jb) in einem rechten Teil (1R) der medizinischen Kopfbedeckung (1) miteinander verbunden sind, wobei der linke Teil (1L) und der rechte Teil (1R) innerhalb der medizinischen Kopfbedeckung (1) einander gegenüberliegend angeordnet sind, wobei die starren elastischen Elemente (12; 12a, 12b, 12c) jeweils einen Gleitmechanismus (15) zur individuellen Einstellung der Länge des jeweiligen starren elastischen Elements (12; 12a, 12b, 12c) zur flexiblen Anpassung des Innenraums (S) an die Form und Größe des Kopfes des Benutzers umfassen, und
wobei die starren elastischen Elemente (12; 12a, 12b, 12c) in dem jeweiligen Verbindungsbereich (J; Ja, Jb) über Gelenkverbindungen (13; 13a, 13b) miteinander verbunden sind, wobei jede Gelenkverbindung (13; 13a, 13b) ein Ende der mindestens drei starren elastischen Elemente (12; 12a, 12b, 12c) derart aufnimmt, dass jedes der mindestens drei starren elastischen Elemente (12; 12a, 12b, 12c) in dem jeweiligen Verbindungsbereich (J; Ja, Jb) gegenüber den anderen schwenken kann, um eine Anpassung an unterschiedliche Formen des Kopfes des Benutzers zu ermöglichen.

2. Medizinische Kopfbedeckung (1) nach Anspruch 1, wobei die starren elastischen Elemente (12; 12a, 12b, 12c) jeweils mindestens eine Blattfeder (B) mit einer Neutralstellung oder einem freien Zustand in einer halbkreisförmigen Form umfassen oder daraus bestehen, wobei die mindestens eine Blattfeder (B) vorzugsweise aus Metall hergestellt ist.

3. Medizinische Kopfbedeckung (1) nach Anspruch 2, wobei der jeweilige Gleitmechanismus (15) ein Führungsteil (15b) umfasst, wobei die mindestens eine Blattfeder (B) zumindest teilweise, z. B. mit ihrem Endabschnitt, beweglich in dem Führungsteil (15b) aufgenommen und durch dieses geführt ist, so dass sich die mindestens eine Blattfeder (B) gleitend gegenüber dem jeweiligen Führungsteil (15b) bewegen kann, um ein gleitendes Ausfahren und Einziehen des jeweiligen starren elastischen Elements (12; 12a, 12b, 12c) zu ermöglichen.

4. Medizinische Kopfbedeckung (1) nach Anspruch 3, wobei der jeweilige Gleitmechanismus (15) eine Zugfeder (15a) umfasst, die die mindestens eine Blattfeder (B) und das jeweilige Führungsteil (15b) federnd verbindet und das jeweilige starre elastische Element (12; 12a, 12b, 12c) in eine eingezogene Stellung vorspannt.

5. Medizinische Kopfbedeckung (1) nach einem der vorhergehenden Ansprüche, wobei jedes der mindestens drei starren elastischen Elemente (12; 12a, 12b, 12c) um eine Schwenkachse (A) schwenken kann, die sich senkrecht zu der halbkreisförmigen Erstreckungsrichtung des jeweiligen starren elastischen Elements (12; 12a, 12b, 12c) erstreckt, um eine Anpassung an unterschiedliche Formen des Kopfes des Benutzers zu ermöglichen.

6. Medizinische Kopfbedeckung (1) nach einem der vorhergehenden Ansprüche, wobei jede der Gelenkverbindungen (13; 13a, 13b) einen Vorsprung (13c) mit einer geneigten Oberfläche (13d) umfasst, wobei das jeweilige starre elastische Element (12; 12a, 12b, 12c) ein fingerförmiges Ende (12d) umfasst, das um die Schwenkachse (A) an dem Vorsprung (13c) schwenkbar gelagert ist, so dass das fingerförmige Ende (12d) des jeweiligen starren elastischen Elements (12; 12a, 12b, 12c) auf der geneigten Oberfläche (13d) aufliegt, um die Drehbewegung des jeweiligen starren elastischen Elements (12; 12a, 12b, 12c) um die Schwenkachse (A) zu begrenzen.

7. Medizinische Kopfbedeckung (1) nach einem der vorhergehenden Ansprüche, wobei die Funktionsmodule (F) Diodenpatches (2) sind, die jeweils mindestens drei, vorzugsweise sechs, Diodensysteme (3) mit Dioden (3a) aufweisen, die Licht entlang einer optischen Achse (O) emittieren, die durch ein Linsensystem (3b) des jeweiligen Diodensystems (3) definiert ist, wobei die Dioden (3a) der Diodensysteme (3) des jeweiligen Diodenpatches (2) vorzugsweise dazu konfiguriert sind, Laserlicht oder LED-Licht mit einer Wellenlänge von ca. 650 nm zur therapeutischen Behandlung androgenetischer Alopezie oder zwischen 800 nm und 1100 nm zur therapeutischen Stimulation des Gehirns mittels transkranieller Lasertherapie zu emittieren, um eine Lichttherapie-Kopfbedeckung (1a) als medizinische Kopfbedeckung (1) bereitzustellen.

8. Medizinische Kopfbedeckung (1) nach Anspruch 7, wobei die Diodensysteme (3) der Diodenpatches (2) von einem vorderen Gehäuseteil (2a) des jeweiligen Diodenpatches (2) umgeben sind, wobei das vordere Gehäuseteil (2a) mindestens eines der Diodenpatches (2) gebildet ist durch
- mindestens drei, vorzugsweise sechs, transparente fingerförmige Abschnitte (2c2), die voneinander beabstandet sind, wobei jeder transparente fingerförmige Abschnitt (2c2) einem der Diodensysteme (3) des jeweiligen Diodenpatches (2) zugeordnet ist und jeder fingerförmige Abschnitt (2c2) in Richtung der optischen Achse (O) des zugeordneten Diodensystems (3) ausgerichtet ist, so dass sich das emittierte Licht durch den fingerförmigen Abschnitt (2c2) ausbreitet und den jeweiligen fingerförmigen Abschnitt (2c2) an dessen als abgerundete Spitze (2d) ausgebildetem distalen Ende verlässt; oder
- einen flachen Abschnitt (2c1), der sämtliche Diodensysteme (3) des jeweiligen Diodenpatches (2) umgibt und eine flache Oberfläche (2g), insbesondere eine gekrümmte flache Oberfläche (2g), aufweist.

9. Medizinische Kopfbedeckung (1) nach Anspruch 7 oder 8, wobei die Diodenpatches (2) ferner ein rückseitiges Gehäuseteil (2f) umfassen, das die Rückseite des jeweiligen Diodenpatches (2) bildet, wobei das rückseitige Gehäuseteil (2f) ein wärmeleitendes Material, z. B. die Aluminiumlegierung 1050A, enthält oder daraus hergestellt ist.

10. Medizinische Kopfbedeckung (1) nach einem der vorhergehenden Ansprüche, wobei die Funktionsmodule (F) federnd innerhalb von Gruppenelementen (4) zusammengefasst sind, wobei jedes Gruppenelement (4) mindestens drei der Funktionsmodule (F) umfasst, die voneinander beabstandet sind und über ein Stützelement (7) federnd miteinander verbunden sind, wobei das Stützelement (7) mindestens drei federnde Arme (9) aufweist, die in unterschiedliche Richtungen vorstehen, wobei jeder federnde Arm (9) mit einem der mindestens drei Funktionsmodule (F) zum federnden Tragen des jeweiligen Funktionsmoduls (F) verbunden ist.

11. Medizinische Kopfbedeckung (1) nach einem der vorhergehenden Ansprüche, wobei mindestens eines der Befestigungselemente (17), das das jeweilige Funktionsmodul (F) mit der Superstruktur (5), insbesondere mit dem starren elastischen Element (12; 12a, 12b, 12c) der Superstruktur (5), verbindet, eine Schalenstruktur (17b) umfasst, die die bewegliche Aufnahme einer Kugelstruktur (17a) ermöglicht, an der das Funktionsmodul (F) einzeln oder innerhalb einer Gruppe befestigt ist, um eine Winkelverstellung des jeweiligen Funktionsmoduls (F) gegenüber der Superstruktur (5) zu ermöglichen.

12. Medizinische Kopfbedeckung (1) nach einem der vorhergehenden Ansprüche, wobei sie ferner eine elektronische Steuereinheit (ECU) umfasst, die elektronisch mit den Funktionsmodulen (F) verbunden ist, um die Behandlung und/oder Diagnosen des Kopfes des Benutzers über diese Funktionsmodule (F) zu steuern, wobei die elektronische Steuereinheit (ECU) in einem Hauptkörperelement (14) der medizinischen Kopfbedeckung (1), insbesondere einen der Verbindungsbereiche (J) umgebend, angeordnet ist und eine Hauptleiterplatte (20) umfasst, die über Kabel (C2) elektronisch mit den mehreren Funktionsmodulen (F) verbunden ist.

## Revendications

1. Dispositif (1) médical de recouvrement de la tête destiné au traitement et/ou au diagnostic de la tête d'un utilisateur, comprenant :
- des modules fonctionnels (F) destinés au traitement et/ou au diagnostic de la tête de l'utilisateur ; et
- une superstructure (5) comprenant au moins trois éléments (12 ; 12a, 12b, 12c) élastiques rigides de forme essentiellement semi-circulaire, assemblés au niveau d'une zone de jonction (J) de manière à enfermer partiellement un espace intérieur (S) présentant une surface courbe destiné à loger la tête de l'utilisateur, où
--- au moins un élément (12a) élastique rigide avant s'étendant dans une partie avant (1F) du dispositif (1) médical de recouvrement de la tête et au moins un élément (12b) élastique rigide arrière s'étendant dans une partie arrière (1B) du dispositif (1) médical de recouvrement de la tête sont assemblés dans la zone de jonction (J) de manière à former un cercle ou une ellipse, rigide et élastique, autour de l'espace intérieur (S), et
--- au moins un élément (12c) élastique rigide supérieur s'étend dans une partie supérieure (1U) du dispositif (1) médical de recouvrement de la tête et enfermant partiellement l'espace intérieur (S) par le haut,
grâce à quoi les modules fonctionnels (F) sont reliés à la superstructure (5), individuellement ou par groupes, par l'intermédiaire d'éléments de montage (17), soit de manière rigide soit de manière mobile, de telle manière qu'ils sont orientés vers l'espace intérieur (S) afin de s'approcher de, ou de toucher, la tête de l'utilisateur,
**caractérisé en ce que**
les éléments (12 ; 12a, 12b, 12c) élastiques rigides sont chacun assemblés dans une zone (Ja) d'assemblage gauche située dans une partie gauche (1L) du dispositif (1) médical de recouvrement de la tête et dans une zone (Jb) d'assemblage droite située dans une partie droite (1R) du dispositif (1) médical de recouvrement de la tête, respectivement, la partie gauche (1L) et la partie droite (1R) étant situées l'une en face de l'autre au sein du dispositif (1) médical de recouvrement de la tête,
dans lequel les éléments (12 ; 12a, 12b, 12c) élastiques rigides comprennent chacun un mécanisme coulissant (15) destiné à régler individuellement la longueur de l'élément (12 ; 12a, 12b, 12c) élastique rigide respectif afin d'adapter de manière flexible l'espace intérieur (S) à la forme et à la taille de la tête de l'utilisateur, et
dans lequel les éléments (12 ; 12a, 12b, 12c) élastiques rigides sont assemblés dans la zone de jonction (J ; Ja, Jb) respective par l'intermédiaire de liaisons articulées (13 ; 13a, 13b), chaque liaison articulée (13 ; 13a, 13b) recevant une extrémité des au moins trois éléments (12 ; 12a, 12b, 12c) élastiques rigides de sorte que chacun des au moins trois éléments (12 ; 12a, 12b, 12c) élastiques rigides peut pivoter les uns par rapport aux autres dans la zone de jonction (J ; Ja, Jb) respective afin de permettre une adaptation aux différentes formes de la tête de l'utilisateur.

2. Dispositif (1) médical de recouvrement de la tête selon la revendication 1, dans lequel les éléments (12 ; 12a, 12b, 12c) élastiques rigides comprennent chacun ou sont constitués chacun d'au moins un ressort à lame (B) présentant une position neutre ou un état libre sous la forme d'un demi-cercle, dans lequel l'au moins un ressort à lame (B) est de préférence réalisé en métal.

3. Dispositif (1) médical de recouvrement de la tête selon la revendication 2, dans lequel le mécanisme coulissant (15) respectif comprend une partie de guidage (15b), dans lequel l'au moins un ressort à lame (B) est, au moins partiellement, par exemple par sa section d'extrémité, logé de manière mobile et guidé par ladite partie de guidage (15b) de sorte que l'au moins un ressort à lame (B) peut se déplacer de manière coulissante contre la partie de guidage (15b) respective afin de permettre à l'élément (12 ; 12a, 12b, 12c) élastique rigide respectif de s'étendre et se rétracter de manière coulissante.

4. Dispositif (1) médical de recouvrement de la tête selon la revendication 3, dans lequel le mécanisme coulissant (15) respectif comprend un ressort de traction (15a) reliant de manière résiliente l'au moins un ressort à lame (B) et la partie de guidage (15b) respective, précontraignant l'élément (12 ; 12a, 12b, 12c) élastique rigide respectif dans une position rétractée.

5. Dispositif (1) médical de recouvrement de la tête selon l'une quelconque des revendications précédentes, dans lequel chacun des au moins trois éléments (12 ; 12a, 12b, 12c) élastiques rigides peut pivoter autour d'un axe de pivotement (A) s'étendant perpendiculairement à la direction de propagation semi-circulaire de l'élément (12 ; 12a, 12b, 12c) élastique rigide respectif afin de permettre une adaptation aux différentes formes de la tête de l'utilisateur.

6. Dispositif (1) médical de recouvrement de la tête selon l'une quelconque des revendications précédentes, dans lequel chacune des liaisons articulées (13 ; 13a, 13b) comprend une saillie (13c) présentant une surface inclinée (13d), dans lequel l'élément (12 ; 12a, 12b, 12c) élastique rigide respectif comprend une extrémité (12d) en forme de doigt montée de manière pivotante autour de l'axe de pivotement (A) sur la saillie (13c), de sorte que l'extrémité (12d) en forme de doigt de l'élément (12 ; 12a, 12b, 12c) élastique rigide respectif repose sur la surface inclinée (13d) afin de limiter le mouvement de rotation de l'élément (12 ; 12a, 12b, 12c) élastique rigide respectif autour de l'axe de pivotement (A).

7. Dispositif (1) médical de recouvrement de la tête selon l'une quelconque des revendications précédentes, dans lequel les modules fonctionnels (F) sont des patchs à diodes (2), présentant chacun au moins trois, de préférence six, systèmes de diodes (3) avec des diodes (3a) émettant de la lumière le long d'un axe optique (O) qui est défini par un système de lentilles (3b) du système de diodes (3) respectif, dans lequel les diodes (3a) des systèmes de diodes (3) du patch à diodes (2) respectif sont de préférence configurées pour émettre une lumière laser ou une lumière à DEL présentant une longueur d'onde d'environ 650 nm pour le traitement thérapeutique de la pelade, ou entre 800 nm et 1 100 nm pour la stimulation thérapeutique du cerveau par thérapie laser transcrânienne, à des fins de fourniture d'un dispositif (1a) de luminothérapie de recouvrement de la tête en tant que dispositif (1) médical de recouvrement de la tête.

8. Dispositif (1) médical de recouvrement de la tête selon la revendication 7, dans lequel les systèmes de diodes (3) des patchs à diodes (2) sont entourés par une partie (2a) de boîtier avant du patch à diodes (2) respectif, dans lequel ladite partie (2a) de boîtier avant d'au moins l'un des patchs à diodes (2) est constituée de
- au moins trois, de préférence six, parties (2c2) en forme de doigt transparentes espacées les unes des autres, chaque partie (2c2) en forme de doigt transparente étant attribuée à l'un des systèmes de diodes (3) du patch à diodes (2) respectif, et chaque partie (2c2) en forme de doigt étant orientée dans la direction de l'axe optique (O) du système de diodes (3) attribué, de sorte que la lumière émise se propage à travers la partie (2c2) en forme de doigt et sort de la partie (2c2) en forme de doigt respective au niveau de son extrémité distale formée en pointe arrondie (2d) ; ou
- une partie plane (2c1) entourant tous les systèmes de diodes (3) du patch de diodes (2) respectif et présentant une surface plane (2g), en particulier une surface plane courbe (2g).

9. Dispositif (1) médical de recouvrement de la tête selon la revendication 7 ou 8, dans lequel les patchs à diodes (2) comprennent en outre une partie (2f) de boîtier arrière formant la face arrière du patch à diodes (2) respectif, dans lequel la partie (2f) de boîtier arrière comprend ou est réalisée en un matériau thermoconducteur, par exemple un alliage d'aluminium 1050A.

10. Dispositif (1) médical de recouvrement de la tête selon l'une quelconque des revendications précédentes, dans lequel les modules fonctionnels (F) sont combinés de manière résiliente au sein d'éléments de groupe (4), dans lequel chaque élément de groupe (4) comprend au moins trois desdits modules fonctionnels (F) qui sont espacés les uns des autres et qui sont reliés entre eux de manière résiliente par l'intermédiaire d'un élément de support (7), dans lequel l'élément de support (7) présente au moins trois bras résilients (9) faisant saillie dans différentes directions, dans lequel chaque bras résilient (9) est relié à l'un des au moins trois modules fonctionnels (F) afin de supporter de manière résiliente le module fonctionnel (F) respectif.

11. Dispositif (1) médical de recouvrement de la tête selon l'une quelconque des revendications précédentes, dans lequel au moins l'un des éléments de montage (17) reliant le module fonctionnel (F) respectif à la superstructure (5), en particulier à l'élément (12 ; 12a, 12b, 12c) élastique rigide de la superstructure (5), comprend une structure (17b) en forme de coupelle permettant de loger de manière mobile une structure sphérique (17a) à laquelle le module fonctionnel (F) est fixé individuellement ou au sein d'un groupe, pour permettre un réglage angulaire du module fonctionnel (F) respectif par rapport à la superstructure (5).

12. Dispositif (1) médical de recouvrement de la tête selon l'une quelconque des revendications précédentes, comprenant en outre une unité de commande électronique (ECU) reliée électroniquement aux modules fonctionnels (F) pour commander le traitement et/ou le diagnostic de la tête de l'utilisateur par l'intermédiaire de ces modules fonctionnels (F), dans lequel l'unité de commande électronique (ECU) est située dans un élément (14) de corps principal du dispositif (1) médical de recouvrement de la tête, en particulier, entourant l'une des zones de jonction (J), comprenant une carte (20) de circuit imprimé principale, reliée électroniquement aux plusieurs modules fonctionnels (F) par l'intermédiaire de câbles (C2).
